# FASCICULE DE BREVET EUROPEEN

(11) **EP 1 033 946 B1**
(45) Date de publication et mention de la délivrance du brevet: **18.08.2004**
(21) Numéro de dépôt: 98956977.7
(22) Date de dépôt: 27.11.1998
(51) Int. Cl.: A61F 2/00

(54) **DISPOSITIF POUR LA MISE EN PLACE D'UNE PROTHESE DANS LE TRAITEMENT DES HERNIES DE L'AINE PAR VOIE COELIOSCOPIQUE**
VORRICHTUNG ZUM EINFÜHREN EINER PROTHESE ZUR BEHANDLUNG EINES LEISTENBRUCHS BEI EINER ZÖLIOSKOPIE
DEVICE FOR FIXING A PROSTHESIS FOR TREATING GROIN HERNIAS BY CELIOSCOPY

(30) Priorité: 28.11.1997 FR 9715027; 04.02.1998 FR 9801277
(43) Date de publication de la demande: 13.09.2000
(73) Titulaire: Cancel, Richard, 83130 La Garde (FR); Wallace, Richard, 83400 Hyeres (FR); Sassi, Gérard, 83200 Toulon (FR)
(72) Inventeur: Cancel, Richard, 83130 La Garde (FR); Wallace, Richard, 83400 Hyeres (FR); Sassi, Gérard, 83200 Toulon (FR)
(74) Mandataire: Breese, Pierre
(86) Numéro de dépôt international: PCT/FR1998/002559
(87) Numéro de publication internationale: WO 1999/027869

(56) Documents cités:
- EP-A- 0 557 963
- EP-A- 0 557 964
- EP-A- 0 625 334
- EP-A- 0 706 778
- WO-A-92/06638
- US-A- 5 397 332

## Description

La présente invention concerne le traitement des hernies de l'aine par voie coelioscopique.

Plus précisément, elle a pour objet un dispositif pour la mise en place d'une prothèse dans la cavité abdominale dans la mise en oeuvre de ce traitement.

Les hernies de l'aine résultent du passage des organes intra-abdominaux à l'extérieur de la cavité abdominale, traversant la paroi musculaire. Ce passage anormal a pour origine une anomalie qui peut être congénitale ou acquise.

Les hernies de l'aine sont des pathologies très répandues, de sorte que les interventions chirurgicales destinées à réparer ces anomalies sont parmi les plus fréquentes pratiquées à l'heure actuelle.

La réparation des hernies de l'aine pose un double problème, celui de leur fréquence et celui de leur récidive après traitement.

Une technique opératoire imparfaite conduirait à un taux de récidives élevé avec ses conséquences humaines et sociales (interventions chirurgicales répétées ; multiplication des frais engendrés).

De tous temps, on a cherché à améliorer les techniques chirurgicales appliquées au traitement des hernies de l'aine afin de diminuer le nombre des récidives et surtout de pouvoir réinsérer les patients dans leur milieu socio-professionnel dans les meilleurs délais.

Il s'avère que dans la majeure partie des hernies acquises, le traitement le plus fiable soit celui qui permet la mise en place d'une prothèse, c'est-à-dire d'un élément en matériau étranger qui, en provoquant une fibrose intense, sera l'agent de la réparation.

Un tel matériel prothétique pose le problème de sa tolérance mais aussi celui de sa mise en place.

Des progrès considérables ont déjà été faits tant en ce qui concerne le matériau dans lequel l'élément prothétique est réalisé, que la mise en place de ce dernier.

La voie coelioscopique est la technique opératoire la moins invasive possible et la mieux tolérée qui soit.

A présent, le matériel prothétique est mis en place par voie coelioscopique par introduction à l'intérieur d'un trocart. Bien que cela représente un progrès certain tant pour le patient que pour le chirurgien, il est indéniable que les systèmes existants présentent encore des inconvénients.

En effet, la dimension classique des prothèses étant de 13 x 10 cm, il est nécessaire de rouler l'élément prothétique comme une cigarette pour pouvoir l'introduire par l'intermédiaire d'un trocart. Il est fastidieux de dérouler cet élément une fois introduit à l'intérieur de la cavité abdominale et ensuite de le maintenir en bonne position pour pouvoir l'agrafer. Le but de l'invention est de mettre au point un matériel facilitant l'introduction, la mise en place et l'agrafage d'un tel élément à l'intérieur de la cavité abdominale.

Ce but est atteint selon l'invention qui fournit un matériel permettant d'introduire, avec une facilité accrue, une prothèse du type sus-évoqué, sous forme de plaque, à l'intérieur de la cavité abdominale et de la dérouler automatiquement, de telle sorte que sa mise en place et son agrafage sont plus aisés pour le praticien. Le document **EP0557964A** décrit un dispositif de mise en place en forme de tube en combinaison avec un piston selon le préambule de la **revendication 1**, utilisé dans des procédures endoscopiques. Plus précisément, l'invention a pour objet un dispositif pour la mise en place d'une prothèse selon le préambule de la revendication 1 dans le traitement des hernies de l'aine par coelioscopie, caractérisé en ce qu'il comprend en outre Un ressort présentant deux bras de ressort seulement, solidaire du piston (2).

Le dispositif selon l'invention permet le déploiement de la prothèse sur deux bras de ressort seulement. Ce dispositif est fondamental car il permet la libération progressive de la prothèse au fur et à mesure de l'agrafage. Par ailleurs, au fur et a mesure du retrait du dispositif, la plaque est progressivement dégagée et libérée en bonne place de manière à laisser la possibilité de l'agrafer immédiatement dans sa position définitive, ce ressort est non seulement un dispositif de présentation de l'introduction, mais également un dispositif de mise en place de la prothèse dans sa position définitivement.

Avantageusement, le dispositif selon l'invention permet l'introduction de la plaque du côté Homo latéral par rapport à la hernie à traiter, ce qui laisse un débattement beaucoup plus important par l'agrafeuse qui se trouve du côté controlatéral à la hernie.

Par ailleurs, le dispositif permet le déploiement automatique de la prothèse et sa mise en place en position définitive par un seul mouvement instantané et automatique de pression par le pouce sur le piston comme on le ferait avec une seringue ordinaire.

D'autre part, le dispositif selon l'invention est le seul instrument qui permet tous ces avantages et toutes ces innovations dans un tube de 5 mm de diamètre comprenant la totalité des mécanismes et de la prothèse.

Avantageusement, le tube présente un diamètre extérieur d'environ 3 à 12 mm.

Ce mode de réalisation est une variante préférée, dans la mesure où elle évite la réalisation d'une suture après l'intervention. Par ailleurs, elle permet de mettre en oeuvre des trocards normalisés, ce qui réduit le coût de l'intervention.

Dans les dessins annexés :
La figure 1 représente, de façon schématique, un tube pouvant être utilisé pour l'introducteur selcn l'invention.
Les figures 2a, 2b et 2c représentent, de façon schématique, en vue de face, en coupe verticale à gauche et en vue de dessus, respectivement un poussoir pouvant être utilisé pour l'introducteur selon l'invention.

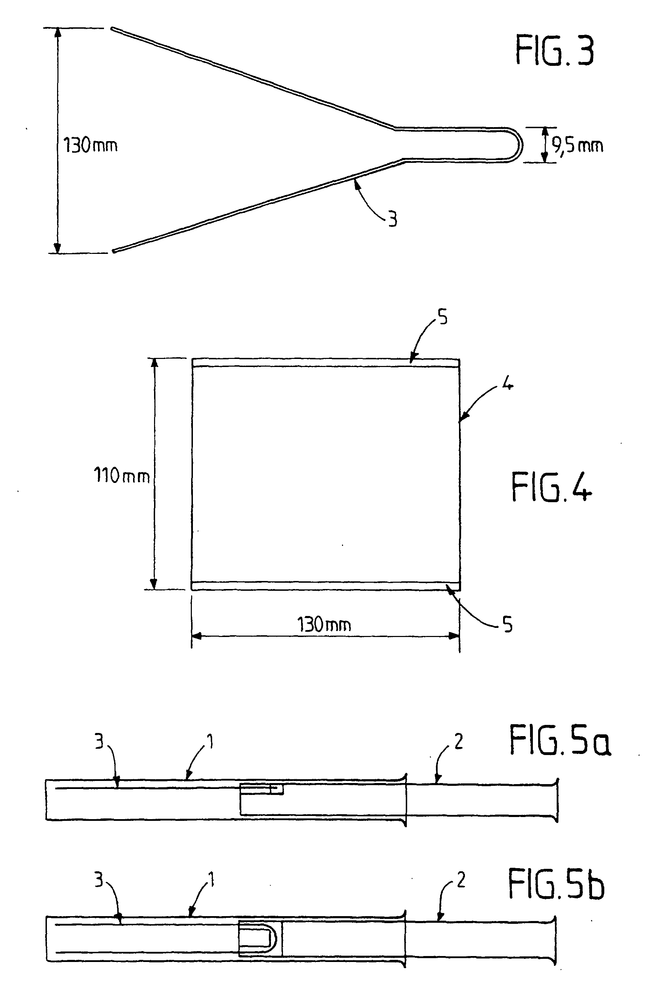

La figure 3 représente un ressort pouvant être utilisé selon l'invention.
La figure 4 représente une plaque carrée souple, destinée à constituer la prothèse et munie d'ourlets dans lesquels peuvent être introduites, de façon amovible, les parties rectilignes du ressort représenté à la figure 3.
Les figures 5a et 5b représentent de façon schématique, en vue. de face et en vue de dessus, respectivement un mode de réalisation du dispositif selon l'invention comprenant les éléments des figures 1 à 3, avant que le piston ne soit enfoncé dans le tube.
La figure 6 représente le dispositif de la figure 5b avec le piston enfoncé.
La figure 7 représente le ressort de la figure 3 avec ses parties rectilignes introduites dans les ourlets de la plaque de la figure 4 partiellement déployée.

L'invention est décrite en détail dans ce qui suit, en référence aux figures.

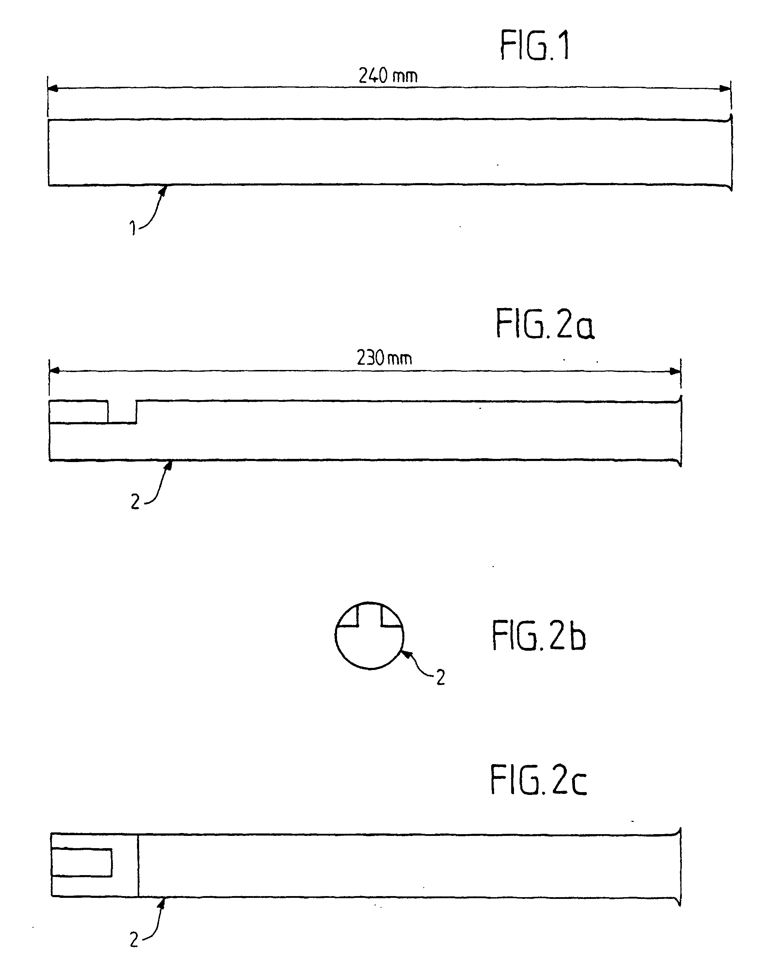

Le tube 1 est en un matériau rigide, notamment en poly(chlorure) de vinyle (PVC), acier inox (304). Son diamètre extérieur est fonction du trocart qui sera utilisé et de son diamètre intérieur destiné à recevoir le piston 2, le ressort 3 et la plaque souple ou prothèse 4 enroulée sur elle-même. De même, la longueur du tube est adaptée pour recevoir ces différents éléments. Typiquement, le tube 1 a une longueur de 240 mm et un diamètre extérieur de 5 mm. Comme montré sur la figure 1, une extrémité du tube 1 présente un moyen de sécurité, par exemple une collerette, empêchant que le tube 1 pénètre trop profondément dans le trocart au moment de l'utilisation.

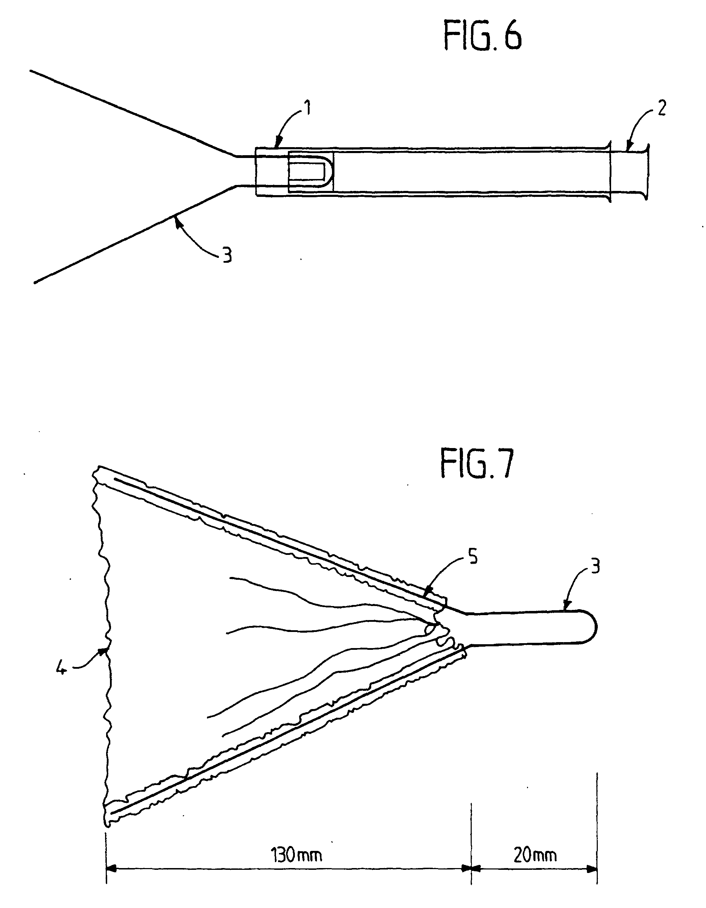

Le poussoir 2 est en un matériau rigide, notamment en poly(chlorure) de vinyle (PVC). Son diamètre extérieur est tel qu'il puisse coulisser sans résistance à l'intérieur du tube 1. Sa longueur est voisine de celle du tube 1. Typiquement, le poussoir 2 a une longueur de 230 mm et un diamètre extérieur de 10 mm. Comme montré sur les figures 2, 5 et 6, une extrémité du poussoir 2 est munie d'un moyen permettant de le pousser ou de le tirer. L'autre extrémité est munie d'un moyen permettant de fixer le ressort 3 pour le rendre solidaire du poussoir 2.

Le ressort 3 est en un métal ou alliage métallique biocompatible, notamment en acier inoxydable et conçu de telle sorte qu'il permette le déploiement de la plaque souple ou prothèse 4 lorsqu'il se détend.

La plaque 4 destinée à constituer la prothèse est en matériau biocompatible suffisamment lacunaire ou poreux pour supporter in vivo une implantation ou croissance tissulaire et suffisamment flexible pour être enroulé sur lui-même et déroulé. Ce matériau est de préférence un matériau textile obtenu par tissage et/ou tricotage et/ou injection d'une résine de polypropylène, de polyamide de polyester etc. La plaque 4 est munie de moyens pour recevoir le ressort 3, tels que par exemple des ourlets 5, des anneaux, des mailles etc. Typiquement, la plaque 4 mesure 110 x 130 mm mais d'autres dimensions peuvent être prévues selon les besoins de l'utilisateur.

Ainsi, en utilisant un même dispositif de mise en place (tube 1 de longueur 150 à 300 mm, de diamètre intérieur 2 à 15 mm et de diamètre extérieur 3 à 12 mm ; typiquement d'environ 5 mm ; piston 2 de longueur 150 à 300 mm et de diamètre extérieur 2 à 15 mm ; parties rectilignes du ressort 3 de 8 à 250 mm), la plaque peut être rectangulaire ou carrée et mesurer par exemple
1. 15 x 13 cm (taille la plus usitée);
2. 15 x 13 cm ;
3. 13 x 13 cm ; ou
4. avoir une autre forme avec découpe.

Les prothèses de types 1 à 3 sont plutôt réservées à la voie coelioscopique transpéritonéale, à l'intérieur de la cavité abdominale, qui est la voie classique de la coeliochirurgie.

La prothèse de type 4 avec découpe préalable de manière à pouvoir faire passer le cordon (élément anatomique qui relie le testicule à l'intérieur de la cavité abdominale en traversant la paroi, ce qui constitue le point de faiblesse) autour de la plaque, est plutôt utilisée par la voie prépéritonéale ou sous- péritonéale selon laquelle on passe entre le péritoine et la paroi musculaire, c'est-à-dire à l'extérieur de la cavité abdominale ou la voie par laparotomie.

L'introducteur qui comprend le tube 1 et le poussoir 2 permet l'injection de la plaque ou prothèse 4 à l'endroit voulu.

Lorsque la plaque 4 est poussée à l'extérieur du tube 1 grâce au poussoir 2, solidaire du ressort 3, elle se déroule à l'extérieur du tube 1, à son extrémité, sous l'effet de la détente du ressort 3 dont elle a elle-même été rendue solidaire avant l'injection.

Une agrafeuse introduite par le trocart contralatéral permet de fixer la plaque 4 tout d'abord au niveau de l'os (ligament de Cooper) et ensuite de l'agrafer vers le haut sans qu'il soit besoin de la manipuler puisqu'elle est maintenue en bonne position au moyen du ressort 3.

L'utilisation selon l'invention d'un tel ressort facilite considérablement le travail du praticien qui n'a plus à dérouler la plaque et à la maintenir ensuite en bonne position pour l'agrafer. Selon la présente invention, c'est le ressort qui assume ces deux fonctions.

Lorsque les deux agrafages expliqués ci-dessus ont été effectués, le dispositif selon l'invention comprenant le tube 1 et le piston 2, solidarisé avec le ressort 3, est progressivement retiré pour dégager la plaque 4.

Enfin, lorsque la plaque est totalement dégagée du dispositif parce qu'il a été entièrement retiré, on agrafe la partie de la plaque qui se trouvait encore dans le tube, dans sa partie verticale.

L'agrafage correct de la plaque ou prothèse 4 en procédant de cette manière permet d'éviter totalement l'agrafage de cette prothèse vers le bas, ce qui pourrait être source de problèmes douloureux post-opératoires.

## Revendications

1. Dispositif pour la mise en place d'une prothèse dans le traitement des hernies de l'aine par coelioscopie, comprenant un introducteur essentiellement constitué d'un tube cylindrique (1) d'un diamètre extérieur, pouvant contenir la prothèse (4) et d'un piston (2) pour l'injection de la prothèse à l'endroit voulu, à partir du tube **caractérisé en ce qu'**il comprend en outre un ressort présentant deux bras de ressort seulement, solidaire du piston (2).

2. Dispositif selon la revendication 1, **caractérisé en ce que** le tube cylindrique (1) est en poly(chlorure) de vinyle.

3. Dispositif selon la revendication 1 ou 2, **caractérisé en ce que** le tube cylindrique (1) présente un moyen de sécurité à l'une de ses extrémités.

4. Dispositif selon l'une quelconque des revendications 1 à 3, **caractérisé en ce que** le piston (2) est en poly(chlorure) de vinyle.

5. Dispositif selon l'une quelconque des revendications 1 à 4, **caractérisé en ce que** le piston (2) est muni d'un moyen permettant de le pousser ou de le tirer.

6. Dispositif selon l'une quelconque des revendications 1 à 5, **caractérisé en ce que** le ressort (3) est en un métal ou alliage métallique biocompatible, notamment en acier inoxydable.

7. Dispositif selon l'une quelconque des revendications 1 à 6, **caractérisé en ce que** :
- le tube (1) a une longueur de 150 à 300 mm, un diamètre intérieur de 2 à 15 mm et un diamètre extérieur de 3 à 12 mm ;
- le piston (2) a une longueur de 150 à 300 mm et un diamètre extérieur de 2 à 15 mm ; et les parties rectilignes du ressort (3) ont une longueur de 8 à 250 mm.

## Patentansprüche

1. Vorrichtung zum Einsetzen einer Prothese zur Behandlung von Leistenbrüchen durch Zölioskopie, umfassend einen Introduktor, der im wesentlichen aus einem zylindrischen Tubus (1) mit einem Außendurchmesser zur Aufnahme der Prothese (4) und einem Kolben (2) zur Injektion der Prothese aus dem Tubus an die gewünschte Stelle besteht, **dadurch gekennzeichnet, dass** sie außerdem eine Feder mit nur zwei mit dem Kolben (2) verbundenen Federarmen umfasst.

2. Vorrichtung nach Anspruch 1, **dadurch gekennzeichnet, dass** der zylindrische Tubus (1) aus Polyvinyl(chlorid) besteht.

3. Vorrichtung nach Anspruch 1 oder 2, **dadurch gekennzeichnet, dass** der zylindrische Tubus (1) an einem seiner Enden ein Sicherheitsmittel aufweist.

4. Vorrichtung nach einem der Ansprüche 1 bis 3, **dadurch gekennzeichnet, dass** der Kolben (2) aus Polyvinyl(chlorid) besteht.

5. Vorrichtung nach einem der Ansprüche 1 bis 4, **dadurch gekennzeichnet, dass** der Kolben (2) mit einem Mittel versehen ist, mit dem er gestoßen oder gezogen werden kann.

6. Vorrichtung nach einem der Ansprüche 1 bis 5, **dadurch gekennzeichnet, dass** die Feder (3) aus einem Metall oder einer biokompatiblen metallischen Verbindung besteht, insbesondere aus rostfreiem Stahl.

7. Vorrichtung nach einem der Ansprüche 1 bis 6, **dadurch gekennzeichnet, dass**:
- der Tubus (1) eine Länge von 150 bis 300 mm hat, einen Innendurchmesser von 2 bis 15 mm und einen Außendurchmesser von 3 bis 12 mm;
- der Kolben (2) eine Länge von 150 bis 300 mm und einen Außendurchmesser von 2 bis 15 mm hat; und die geraden Teile der Feder (3) eine Länge von 8 bis 250 mm haben.

## Claims

1. Device for placement of a prosthesis in the treatment of inguinal hernias by coelioscopy, comprising an insertion instrument consisting essentially of a cylindrical tube (1) with a outside diameter being able to contain the prosthesis (4) and a piston (2) to inject the prosthesis at the required location starting from the tube, **characterised in that** it also comprises a spring with only two spring arms, fixed to the piston (2).

2. Device according to claim 1, **characterised in that** the cylindrical tube (1) is made of polyvinyl chloride.

3. Device according to claim 1 or 2, **characterised in that** the cylindrical tube (1) is provided with a safety mean at one of its ends.

4. Device according to any one of claims 1 to 3, **characterised in that** the piston (2) is made of polyvinyl chloride.

5. Device according to any one of claims 1 to 4, **characterised in that** the piston (2) is provided with a mean of pushing it or pulling it.

6. Device according to any one of claims 1 to 5, **characterised in that** the spring (3) is made of metal or a biocompatible metallic alloy, particularly stainless steel.

7. Device according to any one of claims 1 to 6, **characterised in that**:
- the tube (1) is 150 to 300 mm long, its inside diameter is 2 to 15 mm and its outside diameter is 3 to 12 mm;
- the piston (2) is 150 to 300 mm long and its outside diameter is 2 to 15 mm; and the straight parts of the spring (3) are 8 to 250 mm long.
